(19) Europäisches Patentamt
European Patent Office
Office européen des brevets

(11) **EP 2 211 801 B1**

(12) **EUROPÄISCHE PATENTSCHRIFT**

(45) Veröffentlichungstag und Bekanntmachung des Hinweises auf die Patenterteilung:
**24.04.2013 Patentblatt 2013/17**

(21) Anmeldenummer: **08843390.9**

(22) Anmeldetag: **24.10.2008**

(51) Int Cl.:
***A61F 9/008*** *(2006.01)*

(86) Internationale Anmeldenummer:
**PCT/DE2008/001722**

(87) Internationale Veröffentlichungsnummer:
**WO 2009/056099 (07.05.2009 Gazette 2009/19)**

(54) **VORRICHTUNG ZUR SCHONENDEN LASERTHERAPIE DER RETINA**

APPARATUS FOR GENTLE LASER TREATMENT OF THE RETINA

DISPOSITIF DE TRAITEMENT LASER DOUX DE LA RÉTINE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **31.10.2007 DE 102007052103**

(43) Veröffentlichungstag der Anmeldung:
**04.08.2010 Patentblatt 2010/31**

(73) Patentinhaber: **Medizinisches Laserzentrum Lübeck GmbH 23562 Lübeck (DE)**

(72) Erfinder: **BRINKMANN, Ralf 23562 Lübeck (DE)**

(74) Vertreter: **Eisenführ, Speiser & Partner et al Postfach 31 02 60 80102 München (DE)**

(56) Entgegenhaltungen:
**WO-A-01/91661          WO-A-2005/007002
DE-A1- 10 135 944     DE-A1- 19 932 477
DE-A1-102004 039 194  DE-C1- 4 443 608**

**Beschreibung**

Die Erfindung betrifft eine Laservorrichtung zur Therapie lebenden Gewebes, insbesondere der Netzhaut des lebenden Auges, wobei der Effekt der Laserbestrahlung während der Bearbeitung automatisch überwacht und zur Steuerung des Lasers verwendet wird.

Die Laserphotokoagulation der Netzhaut wird seit mehr als 30 Jahren praktiziert. Ihr therapeutischer Erfolg ist aufgrund vieler Studien für unterschiedliche retinale Erkrankungen unangefochten. Die Anwendungen des Lasers sind sowohl therapeutischer (z.B. Diabetes, Thrombosen des Auges, altersbedingte Makuladegeneration (AMD), als auch vorbeugender Art (z. B. zur Retinopexie). Zur Photokoagulation werden, je nach Erkrankung, zwischen wenigen Einzelexpositionen in der Makula und bis zu 3000 Herden bei panretinaler Photokoagulation appliziert. Je nach Spotgröße wird eine Laserleistung von 100 - 500 mW innerhalb von 50 - 300 ms pro Herd appliziert. Zur Behandlung werden hauptsächlich Laser im grünen Spektralbereich (Ar-Ionen-Laser mit 514 nm oder frequenzverdoppelte Nd-Laser bei 532 nm) eingesetzt; aber auch Laser und Laserdioden im nahen IR.

Die derzeit einzige, praktizierte Dosierung der Laserphotokoagulation besteht in der nachträglichen Kontrolle des ophthalmologischen Erscheinungsbildes der Koagulationsstelle am Fundus. Die dabei auftretende Grau- bzw. Weißfärbung der Netzhaut zeigt eine irreversible thermische Nekrose der neuralen Netzhaut an, die - je nach Stärke und Ausdehnung der Koagulation - die gesamte Netzhaut vom retinalen Pigmentepithel (RPE) über die Photorezeptoren bis zur Nervenfaserschicht reichen und ebenfalls Nekrosen aller Aderhautschichten einschließen kann. Die große räumliche Ausdehnung der Koagulationseffekte resultiert aus der Wärmeleitung von den melaninhaltigen absorbierenden Schichten in die benachbarten Gewebeschichten.

Die panretinale Laserkoagulation der Netzhaut ist die häufigste Anwendung des Lasers in der Augenheilkunde. Hierbei sollen die inneren Schichten der peripheren Netzhaut in 3 - 10 Sitzungen mit bis zu 3000 Laserherden unterschiedlicher Größe thermisch zerstört werden, um das unkontrollierte Gefäßwachstum und die später damit verbundene Erblindung zu verhindern. Bei den meisten Patienten ist die Laserbehandlung äußerst schmerzhaft. Nur eine retrobulbäre Injektion kann zu Schmerzfreiheit führen. Dies hat allerdings eine Ausschaltung der zur Durchführung der Photokoagulation in der Peripherie notwendigen Bulbusbeweglichkeit zur Folge: Bei Überkoagulationen und insbesondere bei Wiederholungsbehandlungen sind zusätzliche thermische Schäden der Ganglionzellschichten zu befürchten, die zu ausgedehnten Gesichtsfelddefekten führen können.

Da die absorbierenden Granula in ihrer örtlichen und räumlichen Dichte beträchtlich variieren, ist es nicht überraschend, dass die histologischen Befunde nach Laserkoagulation selbst bei gleichen Expositionsparametern erheblich unterschiedlich sind. Das Ausmaß der Schädigung hängt im Wesentlichen von der Höhe der laserinduzierten Temperaturerhöhung ab. Diese ist aufgrund unterschiedlicher Pigmentierung und damit Absorption der Netzhaut sowohl inter- als auch intraindividuell praktisch nicht vorhersagbar.

Automatische, temperaturgesteuerte online Dosimetrie bei der Laserbehandlung mit minimal invasiver Schädigung ist ein Wunschziel, das durch die zur Zeit übliche Applikationsmethode in der ophthalmoskopischen Kontrolle nicht möglich ist.

Die Physik bietet verschiedene Techniken zur Temperaturmessung an, jedoch sind fast alle für Messungen am Augenhintergrund praktisch ungeeignet.

Invasive Messtechniken wie zum Beispiel Thermosonden oder temperaturabhängig fluoreszierende Farbstoffe sind zu störend - u. a. wegen Nebenwirkungen - und/oder zu ungenau. Wärmebildkameras sind aufgrund der Absorption der Wärmestrahlung im Auge nicht einsetzbar.

Als Stand der Technik ist die DE 199 32 477 A1 der Anmelderin zu nennen, die bereits einen Therapielaser mit gesteuerter Intensität und/oder Bestrahlungsdauer gearbeitet wird, deren Effekt bei der Bestrahlung lebenden Gewebes detektiert wird.

Autofluoreszenzbasierte Techniken scheinen zwar geeignet zu sein, wie beispielsweise die DE 102 40 109 A1 lehrt, allerdings wird hier eine in der Praxis nicht gegebene gleichmäßige Verteilung der Chromophore vorausgesetzt.

Die Analyse der temperaturabhängigen, thermomechanischen Expansion eines Absorbers und die damit emittierte Druckwelle nach Applikation eines kurzen Laserpulses ist in Sigrist M. W., "Laser Generation of Acoustic Waves in Liquids and Gases", Journal of Applied Physics 60(7):R83-R121, 1986 beschrieben worden. Auf dieser Grundlage entstand die optoakustische Temperaturmessung an der Retina, wie sie in der DE 101 35 944 C2 dargestellt ist. Dabei werden durch zusätzliche, repetitive Bestrahlung mit kurzen Laserpulsen Drucktransienten erzeugt, deren Amplitude sich mit einem im - zur Laserbehandlung ohnehin erforderlichen - Kontaktglas integrierten Ultraschallsensor (z.B. Piezo-Element) aufnehmen lassen. Aus der Amplitude lässt sich die momentane Temperaturerhöhung bestimmen. Hierbei konnte deutlich die Abhängigkeit der Temperatur von der Aderhaut-Perfusion und der Lichtabsorption und damit die absolute Notwendigkeit einer online temperaturbasierten Dosimetrie aufgezeigt werden.

Das Verfahren der DE 101 35 944 C2 wurde bisher für Temperaturmessungen bei der Transpupillaren Thermotherapie (TTT) und der selektiven Retina-Therapie (SRT) eingesetzt. Bei der SRT lassen sich die Therapiepulse selbst zur Bestimmung der Temperatur heranziehen. Die WO 2005/007002 A1 beschreibt ferner Strategien, das optoakustische Signal zur Steuerung des Therapielasers zu benutzen. Allerdings geht die WO 2005/007002 A1 davon aus, dass sich über kurz oder lang mikroskopische Blasen infolge der Lasereinwirkung bilden. Solche Blasen verändern das Verhalten der Drucktransienten signifikant und dienen somit zur Identifizierung der Beschädigungsschwelle, in deren Nähe der Laser arbeiten soll. Dies wird dann durch eine geeignete Rückkopplung realisiert.

Bei der Laserphotokoagulation werden am Augenhintergrund nur Temperaturen von maximal 40-80°C zur Behandlung realisiert. Blasenbildung kann nicht unter 100°C einsetzen, so dass hierin keine Option zur Lasersteuerung liegen kann.

Ziel der Photokoagulation ist die thermische Denaturierung von Proteinen und Gewebe. Insbesondere ist die Abhängigkeit der Ausdehnung und Schadenstiefe von Koagulationen der Netzhaut mit unterschiedlichen Laserparametern experimentell und theoretisch gut untersucht worden (z.B. Birngruber R, Hillenkamp F, Gabel V P., "Experimental studies of laser thermal retinal injury", Health Phys 44(5):519-531, 1983 oder Birngruber R, Hillenkamp F, Gabel V P., "Theoretical investigations of laser thermal retinal injury", Health Phys 48(6):781-796, 1985). Man findet, dass die Schädigung des Gewebes sowohl von der Dauer der Laserbestrahlung als auch unmittelbar - und besonders kritisch - von der während dieser Dauer hervorgerufenen Temperaturerhöhung abhängt. Das Schädigungsintegral $\Omega$ beschreibt dabei eine bestimmte, von Schädigungskriterien und Gewebe abhängige Veränderung, in die der Temperaturverlauf $T(t)$ über die Gesamtdauer der Temperaturerhöhung $t_s$ eingeht.

$$\Omega(t_s) = A \cdot \int\limits_0^{ts} dt \cdot T(t) \cdot e^{-\frac{\Delta E}{k \cdot T(t)}}.$$

Die Aktivierungsenergie $\Delta E$ und der Frequenzfaktor A lassen sich experimentell bestimmen, indem die Schwelle für thermische Schädigung für verschiedene Temperaturerhöhungen und Expositionszeiten bestimmt werden, k bezeichnet die Boltzmann-Konstante. Die Konstanten unterscheiden sich für viele Gewebe. In $\Omega$ geht die Temperatur exponentiell, die Zeit näherungsweise linear ein. Das bedeutet, dass die Auswirkungen einer überhöhten Temperatur weitaus gravierender sein können als die einer zu langen Bestrahlungsdauer. Um stärkere Denaturierung zu beschreiben wird $\Omega$>>1 gewählt (z.B. $\Omega$=100), bleibt man deutlich unterhalb von $\Omega$=1, $\Omega$<<1, so sind keine thermischen Veränderungen zu erwarten.

Es ist die Aufgabe der Erfindung, eine Laservorrichtung zur Netzhautbehandlung anzugeben, die aus optoakustischen Signalen die Expositionsparameter der Laserbestrahlung so steuert, dass eine vorgewählte, an jedem Laserspot gleichartige Veränderung (Schädigung) des Gewebes erzielt wird.

Die Aufgabe wird gelöst durch eine Vorrichtung mit den Merkmalen des Anspruchs 1. Die Unteransprüche geben vorteilhafte Ausgestaltungen an. Vorteilhaft ist dabei insbesondere, dass eine Vorrichtung zur Laserbehandlung lebenden Gewebes mit einem Therapielaser mit einer Quelle für gepulste elektromagnetische Strahlung, einer Steuereinrichtung zur Steuerung der auf dem Gewebe auftreffenden Intensität und/oder der Bestrahlungsdauer des Therapielasers, und einer Detektionseinrichtung zur Detektion optoakustischer Signale, die durch Bestrahlung des lebenden Gewebes mit der gepulsten elektromagnetischen Strahlung ausgelöst werden, geschaffen wird, bei der eine auf die Steuereinrichtung wirkende Auswerteeinrichtung zum Berechnen eines Bewertungsmaßes $B(t)$ für einzelne Laserpulse auf der Grundlage der von der Detektionseinrichtung detektierten optoakustischen Signale und zum Bestimmen einer Fitfunktion $f(t)$ zu einem vorbestimmten Zeitpunkt $\Delta t_1$, bis zu dem noch Keine Gewebeänderungen eingetreten sind, den mittleren Verlauf von $B(t)$ mit von Null verschiedener Steigung für $0 \leq t \leq \Delta t_1$ so approximiert, dass die Laserintensität und/oder die Bestrahlungsdauer des Therapielasers durch die für die Fitfunktion $f(t)$ ermittelten Parameter definiert sind.

Die nachfolgend beschriebene Erfindung ist zur Steuerung in der allgemeinen Therapie - also der Bearbeitung beliebigen lebenden Gewebes - geeignet, wenn während der Bearbeitung ein optoakustisches Signal erzeugt und gemessen wird, das Auskunft über den aktuellen Zustand des Gewebes geben kann. Die Anwendung der Erfindung ist also nicht auf die Ophthalmologie und nicht nur auf Lasertherapien beschränkt, wird aber dort nach heutigem Stand vorrangig gesehen. Im Folgenden wird nur auf die Laserbehandlung des Augenhintergrundes eingegangen.

Der Grundgedanke der Erfindung besteht darin, basierend auf dem in DE 101 35 944 C2 vorgeschlagenen Messaufbau eine irreversible, thermisch bedingte Gewebeveränderung abzuschätzen, bevor sie tatsächlich eintritt. Dies wird durch die fortlaufende Verlaufsüberwachung des optoakustischen Signals und das Realisieren einer Rückkopplung erreicht. Insbesondere ist es bei der Anwendung der Erfindung nicht notwendig, Temperaturen zu bestimmen oder aus den Daten zu berechnen. Lediglich die zeitliche Änderung der Drucktransienten wird erfasst und findet zur Steuerung Verwendung.

Die erfindungsgemäße Vorrichtung weist wenigstens die folgenden Komponenten auf:

● Eine Laserlichtquelle für die Therapiestrahlung (i. F. Therapielaser)

● Eine Steuereinheit für den Therapielaser zur Steuerung der auf dem Gewebe auftreffenden Strahlungsintensität (Strahlungsleistung und Strahldurchmesser),und/oder der Strahlungsdauer (i. F. Bestrahlungsparameter),

● Eine Detektionseinrichtung zur Aufzeichnung von Drucktransienten, die durch gepulste Bestrahlung der Retina entstehen,

● Eine Auswerteeinrichtung mit einem internen Zeitmesser und einem elektronischen Datenspeicher, die die Drucktransienten fortlaufend aufzeichnet, auswertet und durch Schritt haltenden Vergleich mit im Datenspeicher tabellierten Werten entscheidet, welche Bestrahlungsparameter die Steuereinheit einzurichten hat.

Die einzelnen Komponenten sowie die Funktionsweise werden näher erläutert auch anhand der folgenden Figuren:

Fig. 1      zeigt den temperaturabhängigen Verlauf der Arrhenius-Parameter der Netzhaut (Schädigungsintegral $\Omega$, aus der Literatur) für $\Omega$=1 (Kurve: 1, optisch sichtbare Schwelle) und $\Omega$=100 (Kurve: 2, deutliche Koagulation) sowie den errechneten Anstieg der Temperatur bei Bestrahlung eines 200 $\mu$m Spots mit verschiedenen Laserleistungen im grünen Spektralbereich im Zentrum des retinalen Pigmentepithels als stärkstem Absorber (Kurve 3: 20 mW, Kurve 4: 30 mW, Kurve 5: 40 mW) unter Annahme gleicher Absorption.

Fig. 2      zeigt unter der Annahme gleicher Absorption die für die jeweilige Leistung (wie in Fig. 1) bei einem Spotdurchmesser von 200 $\mu$m nötige Bestrahlungszeit bis zum Erreichen der Schwelle ($\Omega$=1).

Fig. 3      zeigt den zu erwartenden Koagulationsbeginn ($\Omega$=1) gegenüber dem Anstieg der Druckamplitude nach 10 ms (Kurve 6, und 7). Lediglich zum Verständnis ist auf der Abszisse zusätzlich der aus Fig. 1 abgeleitete Temperaturanstieg nach 10 ms aufgetragen, da $\Delta\Omega{\sim}\Delta T$.

Der Therapielaser kann als cw-Laser oder als repetierend gepulster Laser ausgebildet sein. Um eine optoakustische Messung überhaupt durchführen zu können, muss notwendig gepulste elektromagnetische Strahlung auf die Netzhaut appliziert werden, vorzugsweise genau auf den vom Therapielaser bearbeiteten Laserspot. Diese Strahlungsimpulse müssen selbst nur für recht geringe Temperaturschwankungen (weniger als 1 °C) sorgen, um zu messbaren Druckwellen zu führen. Deshalb können sie aus einem zweiten, vom Therapielaser völlig unabhängigen Probelaser eingestrahlt werden (vgl. DE 101 35 944 C2), wobei man vorzugsweise beide Strahlen in denselben Strahlengang spiegelt. Tatsächlich ist die Verwendung eines Lasers zur Erzeugung des optoakustischen Signals keineswegs zwingend. Auch eine gepulst betriebene breitbandige Lichtquelle (z.B. SLD, Superlumineszenzdiode) oder ein repetierendes Blitzlicht kommen in Frage (sogar modulierte Mikrowellen- oder Röntgenstrahlung wäre grundsätzlich möglich, wenn auch nicht vorzugsweise am Auge). Die genaue Begrenzung der gepulsten Anregungsstrahlung auf den Therapielaserspot ist günstig, aber durchaus nicht notwendig.

Ein repetierend gepulster Therapielaser (z.B. Pulsleistung 100 W, Pulsdauer um 100 ns, Repetitionsrate um 10 kHz, also on/off-Verhältnis etwa 1:1000) ist aber auch allein ausreichend für die Realisierung der Erfindung. Der Effekt eines solchen "nanopulsing" Lasers auf biologisches Gewebe ist mit dem bei cw-Bestrahlung vergleichbar, jedoch erzeugt jeder einzelne Puls einen kurzzeitigen Temperaturanstieg des Gewebes um Bruchteile eines Grades Celsius. Dies zieht Druckwellen infolge Materialausdehnung nach sich, die ebenfalls mit dem Druckaufnehmer detektiert werden können.

Im Folgenden wird stets davon ausgegangen, dass der Therapielaser dazu ausgebildet ist, repetierend gepulstes Laserlicht auf die Retina zu applizieren, unabhängig davon, ob er dieses aus einer zusätzlichen Probelaserlichtquelle bezieht oder nicht. Insbesondere kommt auch ein cw-Laser mit Chopper in Betracht. Andere Ausgestaltungen des Therapielasers etwa mit Blitzlichtern usw, werden zur Vereinfachung nicht weiter diskutiert. Dem Fachmann wird die Übertragbarkeit der nachfolgend beschriebenen Funktionsweise auf solche Ausgestaltungen ohne weiteres einsichtig sein.

Die Steuereinheit umfasst alle an sich bekannten Mittel, den Strahl des Therapielasers zu verändern, also insbesondere Mittel zur Kontrolle der Pumpleistung oder auch einen akustooptischen Modulator, der einen Anteil des emittierten Laserlichts aus dem Strahlengang zur Retina ablenkt, sowie ansteuerbare Strahloptiken, die den Strahl insbesondere aufweiten können. Laserleistung, Strahldurchmesser und Bestrahlungsdauer sind die zu regelnden Parameter, die den erzielten Gewebeschaden kontrollieren.

Die Detektionseinrichtung ist vorzugsweise als am erforderlichen Kontaktglas angeordneter Ultraschallwandler ausgebildet, der auf Druckschwankungen mit der Erzeugung elektrischer Signale reagiert. Die Signale sind der Druckamplitude proportional und werden der Auswerteeinrichtung zugeführt. An dieser Stelle sei bemerkt, dass es noch andere mögliche Ausbildungen der Detektionseinrichtung gibt, insbesondere solche zur kontaktlosen Messung. Auf diese wird hier nicht näher eingegangen, aber die vorliegende Erfindung sollte nicht als eingeschränkt auf eine bestimmte Art der Messung der Drucktransienten angesehen werden.

Der neue Aspekt der erfindungsgemäßen Vorrichtung liegt in der Art der Auswertung der aufgezeichneten Signale durch die Auswerteeinrichtung. Letztere hat zwei Aufgaben zu erfüllen:

1. Die Drucktransienten werden von der Detektionseinrichtung als Funktionen der Zeit abgetastet und an die Auswerteeinrichtung übermittelt. Die Auswerteeinrichtung misst kontinuierlich die verstrichene Zeit, solange der Therapielaser die Retina bestrahlt. Sie berechnet für jede Drucktransiente, die von einem einzelnen Lichtpuls hervorgerufen wird, eine Hilfsgröße, die hier als Bewertungsmaß bezeichnet werden soll. Als Bewertungsmaß kommen die Maximalamplitude oder die Fläche unter der Drucktransientenkurve (z.B. Absolutintegral) in Betracht, aber auch Phasenverschiebungen und Frequenzänderungen der Drucktransienten geben Auskunft über die Zustandsänderungen des Gewebes infolge der Therapiestrahlungeinwirkung. Es hat sich gezeigt, dass diese Größen im Mittel mit der Temperatur des Augenhintergrundes gut korrelieren, z.B. sind Druck- und Phasenänderungen über kleine Bereiche proportional zu Temperaturänderungen. Da für jeden Lichtpuls ein Wert des Bewertungsmaßes der Drucktransienten gebildet wird, liegt das Bewertungsmaß hiernach als Funktion der Zeit, $B(t)$, in einem ersten Zeitintervall der Dauer $\Delta t_1$ vor. Dieses erste Zeitintervall beginnt bei Einsetzen der Therapiestrahlung (= Beginn der Zeitmessung) und sei als "Anlaufphase" bezeichnet.

2. $B(t)$ ist zunächst gewöhnlich stark verrauscht und wird ggf. von der Auswerteeinrichtung durch eine Glättungsprozedur geglättet, etwa durch eine Fenstermittelung mit einer Fensterbreite sehr viel kleiner als $\Delta t_1$. Die geglättete Funktion $\langle B(t) \rangle$ wird am Ende der Anlaufphase mit einer gängigen Prozedur (z.B. Least Squares o. ä.) durch eine einfache analytische Funktion angefittet, die den zukünftigen Verlauf des Bewertungsmaßes bei Beibehaltung der aktuellen Bestrahlungsparameter durch Extrapolation vorausschätzt. Die Auswerteeinrichtung vergleicht die aufgefundenen Fitparameter für die einfache analytische Funktion mit im internen Datenspeicher tabellierten Daten. Auf der Basis dieses Vergleichs wird dann die Steuereinheit zur Anpassung der Bestrahlungsparameter kontrolliert.

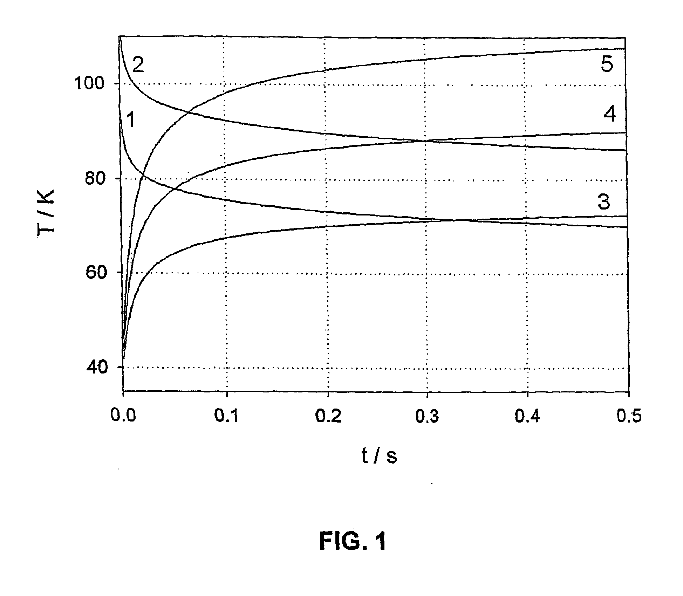

Die Funktionsweise der erfindungsgemäßen Vorrichtung wird im Weiteren näher erläutert. Nach einigen Vorbemerkungen werden konkrete Ausgestaltungen der Erfindung aufgezeigt. In Fig. 1 sind Kurven zur Temperatur-Zeit-Abhängigkeit der Denaturierung von Proteinen in der Retina aus der Literatur (Kurven 1 und 2) wiedergegeben. Beide Kurven zeigen Iso-Linien gleichen Schadensausmaßes (Kurve 1: $\Omega=1$, gerade sichtbare Koagulation; Kurve 2: $\Omega=100$, starke Koagulation) in Abhängigkeit von den gewählten Expositionsparametern (hier: Temperatur und Zeit). Ferner zeigt Fig. 1 den unter Annahme gleicher Absorption errechneten Anstieg der Temperatur bei Bestrahlung eines 200 $\mu$m Spots mit verschiedenen Laserleistungen im grünen Spektralbereich im Zentrum des retinalen Pigmentepithels als stärkstem Absorber (Kurve 3: 20 mW, Kurve 4: 30 mW, Kurve 5: 40 mW). Die Berechnung berücksichtigt keine Veränderung des Gewebes.

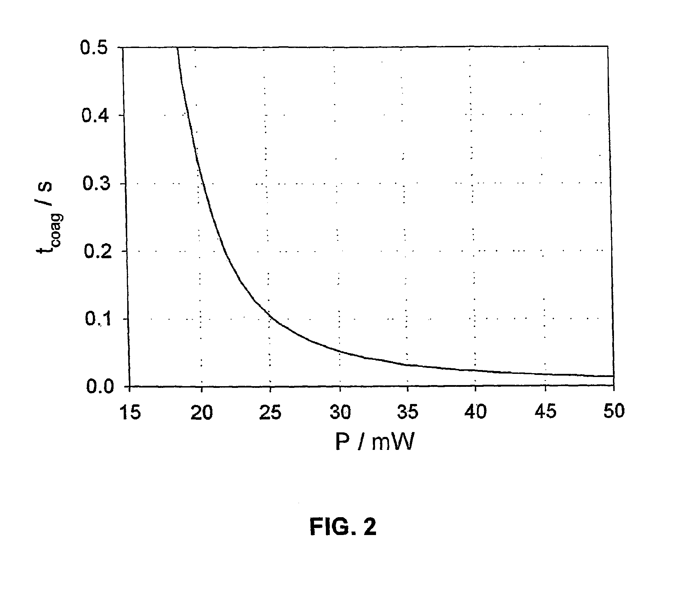

Offensichtlich schneiden sich die $\Omega$-Kurvenschar (1, 2) und die Kurvenschar (3, 4, 5) über kurz oder lang, und insbesondere die Schnittpunkte mit Kurve 1 geben für die verschiedenen Laserleistungen jene Zeitpunkte an, bei denen erstmals Koagulation sichtbar wird. Zur besseren Veranschaulichung zeigt Fig. 2 die Abhängigkeit dieses Zeitpunktes $t_{coag}$ von der Laserleistung als Kurve.

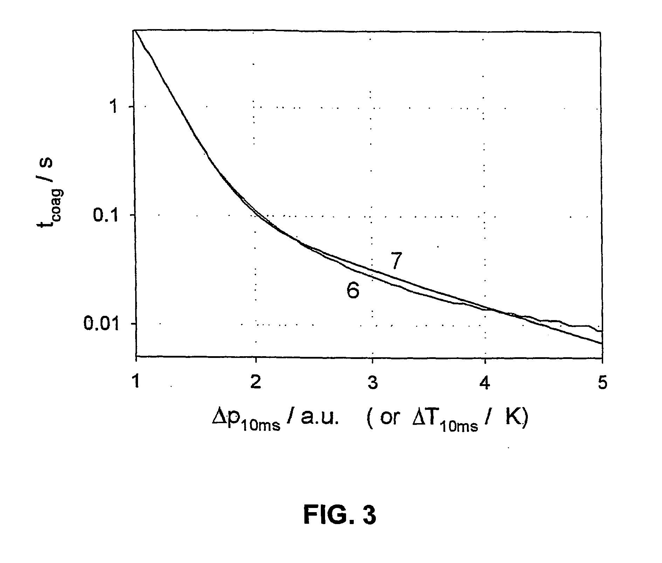

Verschiedene Laserleistungen führen - bei gleicher Absorption - zu unterschiedlichen Temperaturanstiegen kurz nach Einschalten des Therapielasers. Fig. 3 zeigt deshalb alternativ die Zeit $t_{coag}$ als Funktion des Temperaturanstieges (Kurve 6). Da der Grüneisenkoeffizient über kleine Temperaturbereiche sehr gut linear approximiert werden kann, ist in Fig. 3 die Änderung der Druckamplitude $\Delta p$ auf der Abszisse aufgetragen, die direkt messbar ist. (Erinnerung: Die Druckamplitude ist ein mögliches Bewertungsmaß der Drucktransienten.) Eine Umrechnung in Temperaturen ist also nicht notwendig. Selbst wenn man die zweite Nährung (parabolische Entwicklung der Temperatur mit dem Druck) berücksichtigt, so ändern sich die Zeiten bis zur Koagulation nur wenig (Kurve 7).

Der Druckanstieg zu Beginn der Behandlung ist damit ein direktes Maß dafür, wann die Koagulation einsetzt. Die Geschwindigkeit dieses Anstieges ist vor Beginn der Bestrahlung unvorhersehbar, wenn die Materialeigenschaften am gewählten Ort des Laserspots nicht genau bekannt sind (dies ist speziell für Retina-Gewebe immer der Fall).

Aus dem Verlauf des Bewertungsmaßes während der Anfangsphase, in der noch keine Gewebeveränderungen eintreten, erfolgt erfindungsgemäß durch Extrapolation und Schnittpunktsbestimmung mit den vorab bekannten $\Omega$-Kurven, die das Schadensausmaß beschreiben, eine vernünftige Abschätzung der Gesamtzeit bis zur Koagulation. Die $\Omega$-Kurven liegen beispielsweise als Wertetabellen im Datenspeicher der Auswerteeinrichtung vor, die die Fitprozedur vornimmt und den Schnittpunkt der Fitkurve mit einer gewählten $\Omega$-Kurve berechnet. Da diese Berechnung unmittelbar nach der Anfangsphase erfolgt, ergibt sich die Restzeit bis zum Erreichen des gewünschten Gewebeschadens.

In einer ersten Ausführungsform der Erfindung steuert die Auswerteeinrichtung die Steuereinrichtung nach Verstreichen der zuvor berechneten Restzeit an und veranlasst das Abschalten des Therapielasers.

Der behandelnde Arzt kann den Therapielaser vorzugsweise nur manuell i. a. nach dem Wechsel zu einem anderen Laserspot wieder aktivieren. Das Ausmaß des gewünschten Gewebeschadens kann der Arzt vor Behandlungsgewinn durch manuelle Programmierung der Auswerteeinrichtung auswählen. Vorzugsweise stellt er menügeführt einen bestimmten $\Omega$-Wert ein.

Die erste Ausgestaltung der Erfindung geht von zwei Grundvoraussetzungen aus:

1. Es müssen gesicherte Erkenntnisse über den Verlauf der $\Omega$-Kurvenschar, beispielsweise aus der Literatur oder durch eigene - ggf. auch aufwendige - Voruntersuchungen existieren. Dabei ist das im Stand der Technik diskutierte Arrhenius-Modell nicht die einzige Möglichkeit, die Gewebeschädigung zu definieren. Insbesondere kann es für verschiedene Krankheitsbilder oder Therapieansätze zweckmäßig sein, ganz andere Modelle oder neue Wertetabellen zu ermitteln und im Speicher der Auswerteeinrichtung vorzuhalten. Letzten Endes muss die Kurvenschar vor der Therapienutzung der Vorrichtung für das jeweils in Betracht zu ziehende Bewertungsmaß empirisch ausreichend genau bestimmt worden sein.

2. Da die Fitprozedur der Auswerteeinrichtung zur Extrapolation von <B(t)> nach der Anfangsphase dienen soll, muss eine angemessene Fitfunktion vorgegeben werden. Eine physikalisch sinnvolle Wahl ist eine solche, die sicherstellt, dass sich die Fitfiunktion für große Expositionszeit einem endlichen Endwert annähert. Hier wird konkret die Funktion

$$(1) \qquad f(t) = a - b \exp(-\lambda t) \qquad \text{mit } a, b, \lambda \text{ als Fitparametern}$$

vorgeschlagen. Diese beginnt bei t=0 (Start der Therapiestrahlung) beim Startwert a-b, weist eine Anfangssteigung von $b\lambda$ auf und nähert sich allmählich dem Endwert a an. Die Fitparameter sind leicht aus den während der Anfangsphase ermittelten Werten von <B(t)> zu bestimmen, so dass in guter Näherung

$$(2) \qquad <B(t)> \cong f(t) \qquad \text{für } 0 \leq t \leq \Delta t_1$$

gilt. Die Auswerteeinrichtung berechnet nun den Schnittpunkt von f(t) mit der vorgewählten $\Omega$-Kurve aus dem Datenspeicher und berechnet den Zeitpunkt $t_{coag} > \Delta t_1$, an dem der Therapielaser abzuschalten ist. Dieser Zeitpunkt hängt offensichtlich von der Wahl der Fitfunktion f(t) ab, die durchaus auch eine andere Gestalt haben könnte. Beispielsweise käme auch ein Polynom in Betracht. Auch hier mag es günstig sein, für verschiedene Therapiezwecke und/oder Krankheitsbilder verschiedene Fitfunktionen heranzuziehen.

Es bleibt auch bei Verwendung der bis hierher vorgestellten erfindungsgemäßen Vorrichtung eine gewisse Unsicherheit über den tatsächlich erzielten Gewebeschaden bestehen. Diese Unsicherheit ist jedoch gegenüber den Maßnahmen nach Stand der Technik - visuelle Inspektion durch den Arzt - erheblich verringert.

Eine weitere Verbesserung lässt sich mit einer zweiten Ausgestaltung der Erfindung erzielen. Eine optimale Fitfunktion f(t) ist nach dem oben Gesagten höchstens im Einzelfall eindeutig festlegbar. Bei ihrer Wahl kann man dennoch die wahre Entwicklung des Bewertungsmaßes systematisch über- oder unterschätzen, so dass die Therapiestrahlung dann durchweg zu kurz oder zu lang appliziert wird. Dies zieht nach sich, dass die Gewebeschädigungen besonders unterschiedlich ausfallen können zwischen zwei Laserspots, die sich stark in ihrer Absorption - und damit in der Anfangssteigung des Bewertungsmaßes - unterscheiden.

Erfindungsgemäß wird deshalb zusätzlich ein wiederholbarer Mess- und Bewertungsvorgang vorgesehen. Für die während der Anfangsphase ermittelten Fitparameter wird überprüft, ob der Verlauf von f(t) einem vorgegeben Positivkriterium entspricht. Falls ja, wird das Lasern wie zuvor beschrieben fortgesetzt, anderenfalls wird die Laserleistung verändert.

Das Positivkriterium soll insbesondere sein, dass sich die berechnete Fitfunktion über den Zeitbereich der Anfangsphase $\Delta t_1$ vollständig innerhalb eines vorgegebenen Korridors befindet, d.h. es wird nur eine begrenzte Auswahl von Anfangsverläufen erlaubt. Insbesondere sind die Anfangssteigungen (z.B. nach Gleichung (1): $b\lambda$) auf einen bestimmten Wertebereich begrenzt, aber auch das Krümmungsverhalten von f(t) kann so eingeschränkt werden. Die Einschränkung ist insbesondere sinnvoll, um die Bestrahlungsdauern in gewissen Grenzen zu halten. So ist beispielsweise bekannt, dass zu lange Bestrahlungszeiten (etliche 100 ms) die Gefahr von unwillkürlichen Augenbewegungen des Patienten mit sich bringen. Besonders bei der Lasertherapie im Bereich der Makula will man dies vermeiden. Legt die erfindungsgemäße Messung der Anfangssteigung des Bewertungsmaßes indes nahe, dass nur eine sehr kurze Bestrahlungszeit (z.B. < 30 ms) zur Koagulation nötig wäre, so bestünde aufgrund der dann sehr schnellen Erhitzung und beginnender Vaporisation die Gefahr von retinalen Blutungen, wie in der Literatur für Pulsdauern weniger Millisekunden gut dokumentiert ist.

Sofern die berechnete Fitfunktion den Korridor, der vorzugsweise zum Vergleich im Datenspeicher vorgehalten wird z.B. in der Form zweier Limitfunktionen $f_{min}(t)$ und $f_{max}(t)$, zwischen denen sich f(t) bewegen darf, während der Anfangsphase nicht einhält, wird der Therapielaser vorzugsweise deaktiviert bis das Gewebe wieder auf seine Ausgangstemperatur abgekühlt ist. Da die Anfangsphase nur kurz ist, ist dies sehr schnell der Fall, und das Gewebe hat noch keinen Schaden genommen. Die Auswerteeinrichtung gibt der Steuereinrichtung die Anweisung, die Leistung des Therapielasers zu steigern, wenn die Fitfunktion den Korridor nach unten verlässt, oder anderenfalls zu verringern. Das Ausmaß der Leistungsanpassung sollte sich vorzugsweise am Ausmaß der Abweichung zwischen Fitfunktion und Korridor orientieren. Die einfachste zweckmäßige Anpassung besteht in der Multiplikation der Laserleistung mit dem Verhältnis der mittleren gewünschten Steigung des Bewertungsmaßes (die ja vorgegeben ist) zur tatsächlich am Laserspot gemessenen Steigung. Anstelle der Laserleistung kann auch die Strahlungsintensität (Leistung/Fläche) durch entsprechende Strahlaufweitung oder -verengung geändert werden.

Wenn der Therapielaser schließlich wieder aktiviert wird, beginnt der obige Mess- und Bewertungsvorgang am selben Laserspot mit der nunmehr veränderten Leistung von vom. Die Anpassungsschritte werden solange wiederholt, bis das Positivkriterium schließlich erfüllt ist. Das erneute Aktivieren des Therapielasers muss - im Unterschied zur oben genannten manuellen Aktivierung beim Übergang von einem Laserspot zum nächsten - zwischen den verschiedenen Anpassungen der Laserleistung vollautomatisch geschehen. Verschiedene Leistungseinstellungen kann die Vorrichtung in Bruchteilen einer Sekunde vornehmen und an der Retina "ausprobieren". Der behandelnde Arzt sieht die Automatik nicht und hält den Applikator währenddessen nur auf den von ihm gewählten Therapiepunkt gerichtet. Müsste er den Therapielaser selbst aktivieren, würde er durch die Bewegung höchstwahrscheinlich den Laserspot verschieben, und die Anpassung würde ggf. nie gelingen.

Ein Spezialfall der vorangehenden Ausgestaltung mit iterativer Anpassung der Laserintensität (Leistung und/oder Bestrahlungsfläche) ist die Ein-Schritt-Iteration, die besonders erwähnt werden sollte. Hierbei wird bei Feststellen einer Abweichung des Anfangsverlaufs des Bewertungsmaßes vom vorgegebenen Korridor keine Deaktivierung des Therapielasers vorgenommen. Stattdessen erfolgt die Anpassung der Therapielaserleistung - wie beschrieben durch Multiplikation mit einem Verhältniswert - im laufenden Betrieb. Die Zeit $t_{coag}$ bis zum Einsetzen der Koagulation wird hier nicht gesondert berechnet, sondern ergibt sich aus dem vorab bekannten Verlauf der gewünschten Kurve, z.B. in der Mitte des Korridors, d.h. sie ist vorgegeben.

Diese spezielle Ausgestaltung der Erfindung ist gewiss etwas ungenauer als oben beschriebene Vorrichtung mit Iteration, denn sie verzichtet auf die Kontrolle des Erfolges der Steuermaßnahme und auf deren Optimierung. Jedoch hat sie den Vorteil, zu garantieren, dass die Behandlungsdauer eines einzelnen Laserspots der heute üblichen entspricht.

Im Unterschied dazu sorgt die iterativ arbeitende Vorrichtung sicher für eine Annäherung des Bewertungsmaßes an den Sollverlauf bzw. Korridor. Praktisch wird das am aktuellen Laserspot vorliegende Absorptionsvermögen gemessen, und eine vollautomatische Anpassung der Laserleistung bewirkt, dass die Energiedeponierung an allen Laserspots in gleicher Weise, insbesondere mit demselben zeitlichen Verlauf, erfolgt. Hierin wird die derzeit bestmögliche Garantie gesehen, eine gleich bleibende Schädigung während der gesamten Behandlung hervorzurufen.

**Patentansprüche**

1.  Vorrichtung zur Laserbehandlung lebenden Gewebes, mit

    - einem Therapielaser mit einer Quelle für gepulste elektromagnetische Strahlung,
    - einer Steuereinrichtung zur Steuerung der auf dem Gewebe auftreffenden Intensität und/oder der Bestrahlungsdauer des Therapielasers, und
    - einer Detektionseinrichtung zur Detektion optoakustischer Signale, die durch Bestrahlung des lebenden Gewebes mit der gepulsten elektromagnetischen Strahlung ausgelöst werden,
    **gekennzeichnet durch**
    eine auf die Steuereinrichtung wirkende Auswerteeinrichtung
    - zum Berechnen eines Bewertungsmaßes B(t) für einzelne Laserpulse auf der Grundlage der von der Detektionseinrichtung detektierten optoakustischen Signale und
    - zum Bestimmen einer Fitfunktion f(t) zu einem vorbestimmten Zeitpunkt $\Delta t_1$, bis zu dem noch keine Gewebeänderungen eingetreten sind, die den mittleren Verlauf von B(t) mit von Null verschiedener Steigung für $0 \leq t \leq \Delta t_1$ approximiert,

    wobei die Laserintensität und/oder die Bestrahlungsdauer des Therapielasers **durch** die für die Fitfunktion f(t) ermittelten Parameter definiert sind.

**2.** Vorrichtung nach Anspruch 1, **dadurch gekennzeichnet, dass** die Laserintensität und/oder die Bestrahlungsdauer des Therapielasers durch Zusammenwirken der für die Fitfunktion f(t) ermittelten Parameter und einer in einem Datenspeicher abgelegten vorbestimmten Wertetabelle für $F(\Omega, t)$ definiert sind, wobei $\Omega$ ein Maß für die Gewebeschädigung ist.

**3.** Vorrichtung nach Anspruch 2, **dadurch gekennzeichnet, dass** die Steuereinrichtung zum Abschalten des Therapielasers nach der Zeit $t_{coag} > \Delta t_1$, die sich bei Vorgabe einer beabsichtigten Gewebeschädigung $\Omega_0$ aus der Bedingung $f_{(t_{coag})} = F(\Omega_0, t_{coag})$ ergibt, eingerichtet ist.

**4.** Vorrichtung nach Anspruch 2, **dadurch gekennzeichnet, dass** der Datenspeicher Limitfunktionen $f_{min}(t)$ und $f_{max}(t)$ aufweist, und die Auswerteeinrichtung zur unmittelbar nach der Berechnung von f(t) erfolgenden Berechnung, ob $f_{min}(t) \leq f(t) \leq f_{max}(t)$ für alle $0 \leq t \leq \Delta t_1$, eingerichtet ist.

**5.** Vorrichtung nach Anspruch 4, **dadurch gekennzeichnet, dass** die Auswerteeinrichtung zur Berechnung des Verhältnisses von gemittelter Steigung von $f_{min}(t)$ und $f_{max}(t)$ im Intervall $0 \leq t \leq \Delta t_1$ und Steigung von f(t) und zur Weitergabe dieses Verhältnisses als Multiplikator für die Laserintensität an die Steuereinheit eingerichtet ist.

**6.** Vorrichtung nach Anspruch 5, **dadurch gekennzeichnet, dass** die Steuereinrichtung zum Abschalten des Therapielasers nach der Zeit $t_{coag} > \Delta t_1$, die sich als Mittelwert der Schnittpunkte von $f_{min}(t)$ und $f_{max}(t)$ mit $F(\Omega_0, t)$ ergibt, eingerichtet ist.

**7.** Vorrichtung nach Anspruch 4, **dadurch gekennzeichnet, dass** die Steuereinrichtung zum Abschalten des Therapielasers eingerichtet ist, wenn nicht $f_{min}(t) \leq f(t) \leq f_{max}(t)$ für alle $0 \leq t \leq \Delta t_1$ ist.

**8.** Vorrichtung nach Anspruch 7, **dadurch gekennzeichnet, dass** die Auswerteeinrichtung zum automatischen Einschalten des Therapielasers mit verringerter bzw. erhöhter Intensität, falls $f(\Delta t_1) > f_{max}(\Delta t_1)$ bzw. $f(\Delta t_1) < f_{min}(\Delta t_1)$, und zum Wiederholen der Fitprozedur eingerichtet ist.

**9.** Vorrichtung nach Anspruch 1, **dadurch gekennzeichnet, dass** das Bewertungsmaß die Maximalamplitude des optoakustischen Signals ist.

**10.** Vorrichtung nach Anspruch 1, **dadurch gekennzeichnet, dass** das Bewertungsmaß das Integral des Absolutbetrags des optoakustischen Signals ist.

**11.** Vorrichtung nach Anspruch 1, **dadurch gekennzeichnet, dass** das Bewertungsmaß die Phasenverschiebung der optoakustischen Signale zueinander ist.

**12.** Vorrichtung nach Anspruch 1, **dadurch gekennzeichnet, dass** das Bewertungsmaß das Frequenzspektrum des optoakustischen Signals und dessen Änderung darstellt.

**13.** Vorrichtung nach einem der vorangehenden Ansprüche, **dadurch gekennzeichnet, dass** der Therapielaser als ein repetierend gepulster Laser mit einer Pulsdauer im Bereich von ca. 1 ns bis ca. 10 $\mu$m bei einer Repetitionsrate > 100 Hz ausgebildet ist.

**14.** Vorrichtung nach einem der Ansprüche 2 bis 13, **dadurch gekennzeichnet, dass** die Wertetabelle $F(\Omega, t)$ aus empirischen Befunden für das in der Therapie zu verwendende Bewertungsmaß in einer Voruntersuchung bestimmt ist.

**15.** Vorrichtung nach einem der Ansprüche 2 bis 14 **gekennzeichnet durch** Mittel zur Auswahl einer von mehreren im Datenspeicher vorliegenden Wertetabellen $F(\Omega, t)$.

**16.** Vorrichtung nach einem der Ansprüche 2 bis 15 **gekennzeichnet durch** Mittel zur Vorgabe der beabsichtigten Gewebeschädigung $\Omega_0$.

**17.** Vorrichtung nach einem der vorangehenden Ansprüche **gekennzeichnet durch** Mittel zur Auswahl der Fitfunktion f(t), der Fitparameter und der Zeit $\Delta t_1$.

**18.** Vorrichtung nach Anspruch 17, **dadurch gekennzeichnet, dass** $f(t) = a - b \exp(-\lambda, t)$ und a, b, $\lambda$ als Fitparameter

auswählbar sind.

19. Vorrichtung nach einem der vorangehenden Ansprüche, **dadurch gekennzeichnet, dass** Intervallgrenzen für die Fitparameter auswählbar sind.

20. Vorrichtung nach einem der Ansprüche 4 bis 8, **dadurch gekennzeichnet, dass** die Limitfunktionen in Verbindung mit der Auswahl der Fitfunktion auswählbar sind.

**Claims**

1. Apparatus for laser treatment of living tissue, comprising

   - a therapy laser having a source of pulsed electro-magnetic radiation,
   - a control device for controlling the intensity impinging on the tissue and/or the radiating period of the therapy laser, and
   - a detection device for detecting optoacoustic signals triggered by radiating the living tissue with the pulsed electro-magnetic radiation,
   **characterized by**
   an evaluation device acting on the control device
   - for calculating an evaluation amount B(t) for single laser pulses on the basis of the optoacoustic signals detected by the detecting device, and
   - for determining a fitting function f(t) at a predetermined time $\Delta t_1$ until which no tissue modifications have been occurred, the fitting function approximating the mean course of B(t) with a non-zero slope for $0 \leq t \leq \Delta t_1$,

   wherein the laser intensity and/or the radiating period of the therapy laser being defined by parameters determined for the fitting function f(t).

2. Apparatus according to claim 1, **characterized in that** the laser intensity and/or the radiating period of the therapy laser are defined by cooperation of the parameters determined for the fitting function f(t) and a predetermined lookup table for $F(\Omega, t)$, wherein $\Omega$ being a measure for the tissue damage.

3. Apparatus according to claim 2, **characterized in that** the control device is configured to deactivate the therapy laser after the time $t_{coag} > \Delta t_1$ resulting from the condition $f(t_{coag}) = F(\Omega_o, t_{coag})$ with precept of a intended tissue damage $\Omega_o$.

4. Apparatus according to claim 2, **characterized in that** the data storage has limiting functions $f_{min}(t)$ and $f_{max}(t)$, and that the evaluation device is configured to calculate whether $f_{min}(t) \leq f(t) \leq f_{max}(t)$ is valid for all $0 \leq t \leq \Delta t_1$ directly after calculating f(t).

5. Apparatus according to claim 4, **characterized in that** the evaluation device is configured to calculate the ratio of mean slope of $f_{min}(t)$ and $f_{max}(t)$ in the interval $0 \leq t \leq \Delta t_1$ and slope of f(t), and to deliver the ratio to the control device as a multiplier for the laser intensity.

6. Apparatus according to claim 5, **characterized in that** the control device is configured to deactivate the therapy laser after the time $t_{coag} > \Delta t_1$ resulting from a mean value of the intersecting points of $f_{min}(t)$ and $f_{max}(t)$ with $F(\Omega_o, t)$.

7. Apparatus according to claim 4, **characterized in that** the evaluation device is configured to deactivate the therapy laser if $f_{min}(t) \leq f(t) \leq f_{max}(t)$ is not valid for all $0 \leq t \leq \Delta t_1$.

8. Apparatus according to claim 7, **characterized in that** the evaluation device is configured to automatically activate the therapy laser with decreased or increased intensity if $f(\Delta t_1) > f_{max}(\Delta t_1)$ or $f(\Delta t_1) < f_{min}(\Delta t_1)$, and to repeat the fitting procedure.

9. Apparatus according to claim 1, **characterized in that** the evaluation amount being the maximum amplitude of the optoacoustic signal.

10. Apparatus according to claim 1, **characterized in that** the evaluation amount being the integral of the absolute value of the optoacoustic signal.

11. Apparatus according to claim 1, **characterized in that** the evaluation amount being the phase shift of the optoacoustic signals to each other.

12. Apparatus according to claim 1, **characterized in that** the evaluation amount represents the frequency spectrum of the optoacoustic signal and variation of the same.

13. Apparatus according to one of the preceding claims, **characterized in that** the therapy laser is provided as a repetitive pulse laser having a pulse period in the region of 1 ns to about 10 $\mu$m and a repetition rate > 100 Hz.

14. Apparatus according to one of the claims 2 to 13, **characterized in that** the lookup table $F(\Omega, t)$ determined from empirical results is designated for the evaluation amount to be used in the therapy in a pre-examination.

15. Apparatus according to one of the claims 2 to 14, **characterized by** means for selecting one of a plurality lookup tables $F(\Omega, t)$ available in the data storage.

16. Apparatus according to one of the claims 2 to 15, **characterized by** means for predetermining the intended tissue damage $\Omega_o$.

17. Apparatus according to one of the preceding claims, **characterized by** means for selecting the fitting function f(t), the fitting parameters and the time $\Delta t_1$.

18. Apparatus according to claim 17, **characterized in that** $f(t) = a - b \exp(-\lambda t)$ and a, b, $\lambda$ are selectable as fitting parameters.

19. Apparatus according to one of the preceding claims, **characterized in that** the interval boundaries for the fitting parameters are selectable.

20. Apparatus according to one of the claims 4 to 8, **characterized in that** the limiting functions are selectable in connection with the selection of the fitting function.


**Revendications**

1. Dispositif de traitement au laser de tissu vivant, comprenant,

   - un laser de thérapie ayant une source de rayonnement électromagnétique pulsé,
   - un dispositif de commande pour la commande de l'intensité incidente sur le tissu et/ou de la durée d'exposition au laser de thérapie, et
   - un dispositif de détection pour la détection de signaux opto-acoustiques qui sont déclenchés par l'exposition du tissu vivant au rayonnement électromagnétique pulsé,
   **caractérisé par**
   un dispositif d'évaluation agissant sur le dispositif de commande
   - pour le calcul d'une grandeur B(t) d'évaluation des impulsions laser individuelles sur la base des signaux opto-acoustiques détectés par le dispositif de détection et
   - pour la détermination d'une fonction f(t) de fit à un instant $\Delta t_1$ déterminé à l'avance jusqu'auquel il ne s'est pas encore produit de modification du tissu, fonction qui donne une approximation de la courbe moyenne de B(t) avec une pente différente de $0 \leq t \leq \Delta t_1$,

   l'intensité laser et/ou la durée d'exposition au laser de thérapie étant définie par les paramètres déterminés pour la fonction f(t) de fit.

2. Dispositif suivant la revendication 1, **caractérisé en ce que** l'intensité laser et/ou la durée d'exposition au laser de thérapie sont définies par coopération des paramètres déterminés pour la fonction f(t) de fit et par une table de valeur pour $F(\Omega,t)$ déterminée à l'avance et mise dans une mémoire de données, $\Omega$ étant une mesure la lésion du tissu.

3. Dispositif suivant la revendication 2, **caractérisé en ce que** le dispositif de commande est conçu pour arrêter le laser de thérapie après le temps $t_{coag} > \Delta t_1$ qui, dans la prescription d'une lésion $\Omega 0$ de tissu envisagée résulte de la condition $f(t_{coag}) = F(\Omega 0, t_{coag})$.

4. Dispositif suivant la revendication 2, **caractérisé en ce que** la mémoire de données a des fonctions $f_{min}(t)$ et $f_{max}(t)$ limites et le dispositif d'évaluation est conçu pour le calcul, s'effectuant immédiatement après le calcul de $f(t)$, du point de savoir si $f_{min}(t) \leq f(t) \leq f_{max}(t)$ pour tout $0 \leq t \leq \Delta t_1$.

5. Dispositif suivant la revendication 4, **caractérisé en ce que** le dispositif d'évaluation est conçu pour le calcul du rapport de la pente moyenne de $f_{min}(t)$ et $f_{max}(t)$ dans l'intervalle $0 \leq t \leq \Delta t_1$ et de la pente $f(t)$ et pour la transmission à l'unité de commande de ce rapport comme multiplicateur pour l'intensité du laser.

6. Dispositif suivant la revendication 5, **caractérisé en ce que** le dispositif de commande est conçu pour arrêter le laser de thérapie après le temps $t_{coag} > \Delta t_1$, qui résulte de la valeur moyenne des points d'intersection de $f_{min}(t)$ et $f_{max}(t)$ avec $F(\Omega_0, t)$.

7. Dispositif suivant la revendication 4, **caractérisé en ce que** le dispositif de commande est conçu pour arrêter le laser de thérapie si $f_{min}(t) \leq f(t) \leq f_{max}(t)$ n'est pas vrai tout $0 \leq t \leq \Delta t_1$.

8. Dispositif suivant la revendication 7, **caractérisé en ce que** le dispositif d'évaluation est conçu pour le branchement automatique du laser de thérapie à une intensité diminuée ou augmentée si $f(\Delta t_1) > f_{max}(\Delta t_1)$ $f(\Delta t_1) < f_{min}(\Delta t_1)$ et pour reprendre la procédure de fit.

9. Dispositif suivant la revendication 1, **caractérisé en ce que** la grandeur d'évaluation est l'amplitude maximum du signal opto-acoustique.

10. Dispositif suivant la revendication 1, **caractérisé en ce que** la grandeur d'évaluation est l'intégrale de la valeur absolue du signal opto-acoustique.

11. Dispositif suivant la revendication 1, **caractérisé en ce que** la grandeur d'évaluation est le déphasage des signaux opto-acoustiques entre eux.

12. Dispositif suivant la revendication 1, **caractérisé en ce que** la grandeur d'évaluation représente le spectre de fréquence du signal opto-acoustique et sa modification.

13. Dispositif suivant l'une des revendications précédentes, **caractérisé en ce que** le laser de thérapie est constitué sous la forme d'un laser pulsé à répétition ayant une durée d'impulsion dans la plage d'environ 1 ns à environ 10 $\mu$m pour une fréquence de répétition > 100 Hz.

14. Dispositif suivant l'une des revendications 2 à 13, **caractérisé en ce que** la table $F(\Omega, t)$ de valeur est définie dans une étude préalable à partir de données empiriques pour la grandeur d'évaluation utilisée dans la thérapie.

15. Dispositif suivant l'une des revendications 2 à 14, **caractérisé par** des moyens de sélection d'une parmi plusieurs tables $F(\Omega, t)$ de valeurs présentes dans la mémoire de données.

16. Dispositif suivant l'une des revendications 2 à 15, **caractérisé par** des moyens de prescription de la lésion $(\Omega_0)$ de tissu envisagée.

17. Dispositif suivant l'une des revendications précédentes, **caractérisé par** des moyens de sélection de la fonction $f(t)$ de fit, des paramètres de fit et du temps $(\Delta t_1)$.

18. Dispositif suivant la revendication 17, **caractérisé en ce que** $f(t)=a-b\exp(-\lambda t)$ et $a$, $b$, $\lambda$ peuvent être sélectionnés comme paramètres de fit.

19. Dispositif suivant l'une des revendications précédentes, **caractérisé en ce que** des limites d'intervalle pour les paramètres de fit peuvent être sélectionnées.

20. Dispositif suivant l'une des revendications 4 à 8, **caractérisé en ce que** les fonctions limites peuvent être sélectionnées en liaison avec la sélection de la fonction de fit.

**FIG. 1**

**FIG. 2**

**FIG. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19932477 A1
- DE 10240109 A1
- DE 10135944 C2
- WO 2005007002 A1

**In der Beschreibung aufgeführte Nicht-Patentliteratur**

- **BIRNGRUBER R ; HILLENKAMP F ; GABEL V P.** Experimental studies of laser thermal retinal injury. *Health Phys,* 1983, vol. 44 (5), 519-531
- **BIRNGRUBER R ; HILLENKAMP F ; GABEL V P.** Theoretical investigations of laser thermal retinal injury. *Health Phys,* 1985, vol. 48 (6), 781-796